# EUROPEAN PATENT SPECIFICATION

(11) **EP 3 020 273 B1**
(45) Date of publication and mention of the grant of the patent: **04.07.2018**
(21) Application number: 14193488.5
(22) Date of filing: 17.11.2014
(51) Int. Cl.: A01K 67/027

(54) **A METHOD OF PRODUCING BIOTECHNOLOGICAL DRUGS USING TRANSGENIC BOVINE ANIMALS**
VERFAHREN ZUR HERSTELLUNG BIOTECHNOLOGISCHER ARZNEIMITTEL UNTER VERWENDUNG TRANSGENER RINDER
PROCÉDÉ DE PRODUCTION DE MÉDICAMENTS BIOTECHNOLOGIQUES À L'AIDE DE BOVINS TRANSGÉNIQUES

(43) Date of publication of application: 18.05.2016
(73) Proprietor: Competence Centre on Health Technologies, 50410 Tartu (EE)
(72) Inventor: KÕKS, Sulev, 50410 Tartu (EE); PLAAS, Mario, 50410 Tartu (EE); PÄRN, Pille, 50410 Tartu (EE); IVASK, Marilin, 50410 Tartu (EE); NÕMM, Monika, 50410 Tartu (EE); KURÕKIN, Jevgeni, 50410 Tartu (EE); MEIER, Riho, 50410 Tartu (EE); JAAKMA, Ülle, 50410 Tartu (EE); REIMANN, Ene, 50410 Tartu (EE); LILLEOJA, Rutt, 50410 Tartu (EE); TAGOMA, Aili, 50410 Tartu (EE)
(74) Representative: Sarap, Margus

(56) References cited:
- EP-A2- 1 217 072
- WO-A1-92/03917
- WO-A1-94/08024
- SALAMONE D ET AL: "High level expression of bioactive recombinant human growth hormone in the milk of a cloned transgenic cow", JOURNAL OF BIOTECHNOLOGY, ELSEVIER SCIENCE PUBLISHERS, AMSTERDAM, NL, vol. 124, no. 2, 13 July 2006 (2006-07-13) , pages 469-472, XP024956745, ISSN: 0168-1656, DOI: 10.1016/J.JBIOTEC.2006.01.005 [retrieved on 2006-07-13]
- BIN YANG ET AL: "Characterization of Bioactive Recombinant Human Lysozyme Expressed in Milk of Cloned Transgenic Cattle", PLOS ONE, vol. 6, no. 3, 16 March 2011 (2011-03-16), page e17593, XP055193395, DOI: 10.1371/journal.pone.0017593

### SEQUENCE LISTING

<110> Competence Centre on Health Technologies
<120> A method of producing biotechnological drugs using transgenic bovine
<130> 450P1 EP
<160> 8
<170> BiSSAP 1.3
<210> 1
   <211> 15
   <212> PRT
   <213> Capra hircus
<400> 1
   Met Lys Val Leu Ile Leu Ala Cys Leu Val Ala Leu Ala Ile Ala
   1               5                   10                  15
<210> 2
   <211> 15988
   <212> DNA
   <213> Artificial Sequence
<220>
   <223> Artifical Sequence
<400> 2
   gggcgaattg gccaagtcgg ccgagctcga attgcgccgg ccaggcgcca gaaagcttcc      60
   tgcaggatct agagtcagct tctgatggaa ttagaacttg gcaaaacaat actgagaatg     120
   aagtgtatgt ggaacagagg ctgctgatct cgttcttcag gctatgaaac tgacacattt     180
   ggaaaccaca gtacttagaa ccacaaagtg ggaatcaaga gaaaaacaat gatcccacga     240
   gagatctata gatctataga tcatgagtgg gaggaatgag ctggccctta atttggtttt     300
   gcttgtttaa attatgatat ccaactatga aacattatca taaagcaata gtaaagagcc     360
   ttcagtaaag agcaggcatt tatctaatcc caccccaccc ccacccccgt agctccaatc     420
   cttccattca aaatgtaggt actctgttct cacccttctt aacaaagtat gacaggaaaa     480
   acttccattt tagtggacat ctttattgtt taatagatca tcaatttctg cagacttaca     540
   ggacggatcg atcccctcag aagaactcgt caagaaggcg atagaaggcg atgcgctgcg     600
   aatcgggagc ggcgataccg taaagcacga ggaagcggtc agcccattcg ccgccaagct     660
   cttcagcaat atcacgggta gccaacgcta tgtcctgata gcggtccgcc acacccagcc     720
   ggccacagtc gatgaatcca gaaaagcggc cattttccac catgatattc ggcaagcagg     780
   catcgccatg ggtcacgacg agatcatcgc cgtcgggcat gcgcgccttg agcctggcga     840
   acagttcggc tggcgcgagc ccctgatgct cttcgtccag atcatcctga tcgacaagac     900
   cggcttccat ccgagtacgt gctcgctcga tgcgatgttt cgcttggtgg tcgaatgggc     960
   aggtagccgg atcaagcgta tgcagccgcc gcattgcatc agccatgatg gatactttct    1020
   cggcaggagc aaggtgagat gacaggagat cctgccccgg cacttcgccc aatagcagcc 1080
   agtcccttcc cgcttcagtg acaacgtcga gcacagctgc gcaaggaacg cccgtcgtgg 1140
   ccagccacga tagccgcgct gcctcgtcct gcagttcatt cagggcaccg gacaggtcgg 1200
   tcttgacaaa aagaaccggg cgcccctgcg ctgacagccg gaacacggcg gcatcagagc 1260
   agccgattgt ctgttgtgcc cagtcatagc cgaatagcct ctccacccaa gcggccggag 1320
   aacctgcgtg caatccatct tgttcaatgg ccgatcccat attggctgca ggtcgaaagg 1380
   cccggagatg aggaagagga gaacagcgcg gcagacgtgc gcttttgaag cgtgcgagaa 1440
   tgccgggcct cgggaggacc ttcgcgcccg ccccgcccct gagcccgccc ctgagcccgc 1500
   ccccggaccc accccttccc agcctctgag cccagaaagc gaaggagcaa agctgctatt 1560
   ggccgctgcc ccaaaggcct acccgcttcc attgctcagc ggtgctgtcc atctgcacga 1620
   gactagtgag acgtgctact tccatttgtc acgtcctgca cgacgcgagc tgcggggcgg 1680
   gggggaactt cctgactagg ggaggagtag aaggtggcgc gaaggggcca ccaaagaacg 1740
   gagccggttg gcgctaccgg tggatgtgga atgtgtgcga ggccagaggc cacttgtgta 1800
   gcgccaagtg ccagcggggc tgctaaagcg catgctccag actgccttgg gaaaagcgcc 1860
   tcccctaccc ggtagaattg acctgcaggg gccctcgaat cgtggatcca gaattcgtcg 1920
   acctcgactc tagagggaca gccccccccc aaagccccca gggatgtaat tacgtccctc 1980
   ccccgctagg ggcagcagcg agccgcccgg ggctccgctc cggtccggcg ctccccccgc 2040
   atccccgagc cggcagcgtg cggggacagc ccgggcacgg ggaaggtggc acgggatcgc 2100
   tttcctctga acgcttctcg ctgctctttg agcctgcaga cacctggggg gatacgggga 2160
   aaaagcttta ggctgaaaga gagatttaga atgacagaat catagaacgg cctgggttgc 2220
   aaaggagcac agtgctcatc cagatccaac cccctgctat gtgcagggtc atcaaccagc 2280
   agcccaggct gcccagagcc acatccagcc tggccttgaa tgcctgcagg gatggggcat 2340
   ccacagcctc cttgggcaac ctgttcagtg cgtcaccacc ctctggggga aaaactgcct 2400
   cctcatatcc aacccaaacc tcccctgtct cagtgtaaag ccattccccc ttgtcctatc 2460
   aagggggagt ttgctgtgac attgttggtc tggggtgaca catgtttgcc aattcagtgc 2520
   atcacggaga ggcagatctt ggggataagg aagtgcagga cagcatggac gtgggacatg 2580
   caggtgttga gggctctggg acactctcca agtcacagcg ttcagaacag ccttaaggat 2640
   aagaagatag gatagaagga caaagagcaa gttaaaaccc agcatggaga ggagcacaaa 2700
   aaggccacag acactgctgg tccctgtgtc tgagcctgca tgtttgatgg tgtctggatg 2760
   caagcagaag ggtggaagga gcttgcctgg agagatacag ctgggtcagt aggactggga 2820
   caggcagctg gagaattgcc atgtagatgt tcatacaatc gtcaaatcat gaaggctgga 2880
   aagcctccaa gatccccaag accaacccca acccacccac cgtgcccact ggccatgtcc 2940
   ctcagtgcca catccccaca gttcttcatc acctccaggg acggtgaccc ccccacctcc 3000
   gtgggcagct gtgccactgc agcaccgctc tttggagaag gtaaatcttg ctaaatccag 3060
   cccgaccctc ccctggcaca acgtaaggcc attatctctc atccaactcc aggacggagt 3120
   cagtgaggat ggggctctag agggacagcc cccccccaaa gcccccaggg atgtaattac 3180
   gtccctcccc cgctaggggc agcagcgagc cgcccggggc tccgctccgg tccggcgctc 3240
   cccccgcatc cccgagccgg cagcgtgcgg ggacagcccg ggcacgggga aggtggcacg 3300
   ggatcgcttt cctctgaacg cttctcgctg ctctttgagc ctgcagacac ctggggggat 3360
   acggggaaaa agctttaggc tgaaagagag atttagaatg acagaatcat agaacggcct 3420
   gggttgcaaa ggagcacagt gctcatccag atccaacccc ctgctatgtg cagggtcatc 3480
   aaccagcagc ccaggctgcc cagagccaca tccagcctgg ccttgaatgc ctgcagggat 3540
   ggggcatcca cagcctcctt gggcaacctg ttcagtgcgt caccaccctc tgggggaaaa 3600
   actgcctcct catatccaac ccaaacctcc cctgtctcag tgtaaagcca ttcccccttg 3660
   tcctatcaag ggggagtttg ctgtgacatt gttggtctgg ggtgacacat gtttgccaat 3720
   tcagtgcatc acggagaggc agatcttggg gataaggaag tgcaggacag catggacgtg 3780
   ggacatgcag gtgttgaggg ctctgggaca ctctccaagt cacagcgttc agaacagcct 3840
   taaggataag aagataggat agaaggacaa agagcaagtt aaaacccagc atggagagga 3900
   gcacaaaaag gccacagaca ctgctggtcc ctgtgtctga gcctgcatgt ttgatggtgt 3960
   ctggatgcaa gcagaagggg tggaagagct tgcctggaga gatacagctg ggtcagtagg 4020
   actgggacag gcagctggag aattgccatg tagatgttca tacaatcgtc aaatcatgaa 4080
   ggctggaaag cctccaagat ccccaagacc aaccccaacc cacccaccgt gcccactggc 4140
   catgtccctc agtgccacat ccccacagtt cttcatcacc tccagggacg gtgacccccc 4200
   cacctccgtg ggcagctgtg ccactgcagc accgctcttt ggagaaggta aatcttgcta 4260
   aatccagccc gaccctcccc tggcacaacg taaggccatt atctctcatc caactccagg 4320
   aacggagtca gtgaggatgg ggctctagag gatccctcga cctgcaggtc aacggatcac 4380
   aacaaactgg aaaattcttc aagagaagaa taccagacca ccctacctgc ttcctgagaa 4440
   atctgtttgc tgctcagaag caacagttag aaccagacat ggaacaacag actggttcca 4500
   aatcaggaaa ggagtatgtc aaggctgtat atcgtcaccc tgattattta acttatatgc 4560
   atagtacata atacaaaatg ccaggctgga tgaatcgcaa gctggaatca agatttctgg 4620
   gagaaatatc aataaacgag atacaaagat acaccacact tatggcagaa aactaagaag 4680
   aactaaagag cctcttgatg aaagtgaaag aggagagtga aaaagccagc ttaaaaccca 4740
   acattcaaaa tcaagatcat catttcatgg caaataaatg gggaaacaat ggaaacagtg 4800
   agagacttta ttttcttggg ctccaaaatc actgcagatt gtgactacag ccatgattaa 4860
   aagatgcttg ctccttggaa gagaagctat taccaaacta gaaagcatat taaaaagcag 4920
   agacgttact ttgctgacta agttctgtct agtcaaacct atggtttttc cagtagtcat 4980
   atatggatgt gagttgaact ataaagaaag ctgagcacca aagaattgat gcttttgaaa 5040
   tttggtgttg gagaagtctc ttgagagtcc cttgaacctg caaggagatc caaccagtcc 5100
   atcctaaagg aaatcagtcc tgaatattca ttggaaggac tgatgctgaa attgaagatt 5160
   aacgttttgg actcacctaa tgcagaagag ccaactcact agaaaagacc ccatgttggc 5220
   aaaaattgaa gccaggaaga gaagtgaatg acagaggatg agatggttgg atggcatcgt 5280
   tgactgaatg gacatgagtc tgatcaagtt ccgggagaca gcaaaggaca gggctgcctg 5340
   gtctgctgca gtccatgggg ttgcaaagag tcggtctcaa atgagtaact aaacaacaac 5400
   caagcagtag aaaaataaat aaaatttgtc tctgagatct cagtacctct ttctgtgcat 5460
   atccgtctcc tgttattgta ctttgtcttc tgcttgtaat aaagctgtcc tgttagtaaa 5520
   atctgtttgg gtcctctgaa ttcttttagc tatcaaaaat ggaaggtgat tattgtgcaa 5580
   tgtccacctc tgagtaatat acagagaata aaagaaggga gaaattatgt gcaagttctc 5640
   tctcatctcc tgcttctcat ttaaaagatt ctacctcagt gggggctaaa actccacatt 5700
   taacagtagc aaaaaccaat attccatagc ttcttaggaa accatttttt atactcttgt 5760
   atgtaattac attcaagctc aaaagcaaag aagtgattct gcgttggtga aggcccaacc 5820
   atagaaaaga ggaagaaaat aggccacata ctgtgcttcc cccatagctc agttggtaaa 5880
   gaatctacct acaatgcagg aggcctgggc ttgatccctg ggtaagggag atcccctgga 5940
   gaaggaaatg gtaacccact ccagtactct tgcctgtaaa tcccatggac ggaggagcct 6000
   ggcagctaca gccttggggt ggcaagagtt ggacatgatt aacaactaaa ccactgccac 6060
   cactccacat actgagtgct ccccagtggc actagtggta aagaaccacc tgccggtgca 6120
   gaagacatta aagacactgg ctctatccct gcttgggaag tagggaagat cccctagaga 6180
   gggaaatagc aacccactcc agaattcttg cctggaaaat cccatgaatg aagactggcg 6240
   ggctgtagta actggggtca caaagagtta aacatgattt agcaactaaa catcaccaca 6300
   ttaaaaaaat taccaccaaa atagtcatat tccaggctaa ggggaataat agcactagta 6360
   cctgagagaa ctttctcaga ttctctgtca agttcttcct tctctcatat aaccagtagt 6420
   ctagtttacc tcatcagata ttaactactc atcgattcta aattatctaa ttatgggggg 6480
   gggcactaca ttgcattata ttttgtgtcc attgactatc actcaattta tttataaaaa 6540
   attcatccat gttgtttctg tgacagtaac tcattcacat taattgtaat atctcattgc 6600
   attgtatact acaatttatt tatacaaaat actattattc acacttctgt tgattttaat 6660
   ttggaacatc aacaataacg tggctgagaa gcttctttct ttagtatatt gttaaggatt 6720
   tccttgatca agattttacc tacttttctg gtccaattgg tgagagacag tcataaggaa 6780
   atgctgtgtt tattgcacaa tatgtaaagc atcttcctga gaaaataaaa gggaaatgtt 6840
   gaatgggaag gatatgcttt cttttgtatt ccttttctga gaaatcagac tttttcacct 6900
   tggccttggc caccaaaagc taacaaataa aggcatatga agtagccaag gccttttcta 6960
   gttatatcta tgacactgag ttcatttcat catttatttt cctgacttcc tcctgggtcc 7020
   atatgagcag tcttagaatg aatattagct gaataatcca aatacatagt agatgttgat 7080
   ttgggttttc taagcaatcc aagacttgta tgacagtaag atgtattacc atccaacaca 7140
   catctcagca tgatataaat gcaaggtata ttgtgaagaa aaatttttaa ttatgtcaaa 7200
   gtgcttactt tagaaggtca tctatctgtc ccaaagctgt gaatatatat attgaaggta 7260
   atgaatagat gaagctaacc ttgtaaaaat gagtagtgtg aaatacaact acaattatga 7320
   acatctgtca ctaaagaggc aaagaaactt gaagattgct tttgcaaatg ggctcctatt 7380
   aataaaaagt acttttgagg tctggctcag actctattgt agtacttagg gtaagaccct 7440
   cctcctgtat gggctttcat tttctttctt gcttccctca tttgcccttc catgaatact 7500
   agctgataaa cattgactat aaaagatatg aggccaaact tgagctgtcc cattttaata 7560
   aatctgtata aataatattt gttctacaaa agtattatct aaataaatgt tactttctgt 7620
   cttaaaatcc ctcaacaaat ccccactatc tagagaataa gattgacatt ccctggaatc 7680
   acagcatgct ttgtctgcca ttatctgacc cctttctctt tctctcttct cacctccatc 7740
   tactcctttt tccttgcaat tcatgaccca gattcactgt ttgatttggc ttgcatgtgt 7800
   gtgtgctgag ttgcgtctga ctgttatcaa ccccatgaat gatagtccac caggctctac 7860
   tgtccatgaa attttccagt caagaatact ggagtggatt gcatttccta ctccatttga 7920
   ttaatttagt gacttttaaa tttctttttc catattcggg agcctattct tcctttttag 7980
   tctatactct cttcactctt caggtctaag gtatcatcgt gtgcttgtta gcttgttact 8040
   ttctccatta tagcttaagc actaacaact gttcaggttg gcatgaaatt gtgttctttg 8100
   tgtggcctgt atatttctgt tgtgtattag aatttacccc aagatctcaa agacccactg 8160
   aatactaaag agacctcatt gtggttacaa taatttgggg actgggccaa aacttccgtg 8220
   catcccagcc aagatctgta gctactggac aatttcattt cctttatcag attgtgagtt 8280
   attcctgtta aaatgctccc cagaatttct ggggacagaa aaataggaag aattcatttc 8340
   ctaatcatgc agatttctag gaattcaaat ccactgttgg ttttatttca aaccacaaaa 8400
   ttagcatgcc attaaatact atatataaac agccactaaa tcagatcatt atccattcag 8460
   cttctccttc acttcttctc ctctactttg gaaaaaaggt aagaatctca gatataattt 8520
   cagtgtatct gctactcatc tttattttgg actaggttaa aatgtagaaa gaacataatt 8580
   gcttaaaata gatcttaaaa ataagggtgt ttaagataag gtttacacta ttttcagcag 8640
   atatgttaaa aaatagaagt gactataaat acttgataaa aattatagtg actgcaaatg 8700
   ttttaggaat ataataagat ataataacag tggttgctat tttctttagc acaagactag 8760
   ttaacaggct gtattaaaag atcttttctt gaattaaata ttttcaattt gattaaacct 8820
   acctcagcca taaaggcaag cacatttcat ttatactatg gggatttgaa taattattac 8880
   tgaagaagct ctaccaacaa aaagtttata gagctatcat atttagtcaa gagataaaga 8940
   gggttgttag gatatatatg ctatttgaaa ggtatttata aaagaagagt atatttatca 9000
   aaatttctca agaacatcca aatttcaagt ttatcattta tcttacaata tttcaaaaat 9060
   attaaaatag atacatgaaa tacagaagta aattaaagag aaagtatttt acttggtaaa 9120
   aaaattctag gttggacaga gagtgccagg aaacaaaaac aatgaaaaat gtgacctgac 9180
   aggaattata gctcaaagta tagtagtaag taatgaaatg gcttaaaaat tggtatataa 9240
   aatgctagtt ataaaataaa caaaatgcaa taatatcctc cctacatgta atgaattcta 9300
   ggtattatga ttatgctctt ttttgaagtc ttgacaataa aaattttttt agaagtttat 9360
   aggcatcttg aataaagtga aacaaattaa gaattagtat ccatgagaaa aatatagaac 9420
   aattttccta atttagtttg aaaatctggg attgaagatg tgtgtcaaga gatgttggtg 9480
   gcaagaacat ttttttttca agaacttata aaaatgcaac aaaacaaacc atttaataca 9540
   ttttggtcaa aatcaataat gtattttatt ttatgctcca aggagcataa aattggggac 9600
   tgggcaagag aaactgacac cctggtaaat taccaagaga taagtacaca gttactatag 9660
   tagaaaataa gcatagtgta tgatctctaa aattatgtga gacaaaggag agatgacatt 9720
   aggcatgtgg ggatgaagac tgagtagaga agaaacaatc taatcagtcc aagaaaacat 9780
   ctcgatcagt ggaacaaata gaagaaatgc taaaatgaaa cagaagtctt actggaaata 9840
   aaagatatgc ataagacaaa aattcatgaa aatcacttag tttagcagag aaaagataaa 9900
   aataaagtat gaccttcttc atatacattg tttgatcata tgcacctcaa taaaactgag 9960
   tctccaacag aaatgaaaca ttaatatttt gttcactgct ctaatcccag aatctaagcg 10020
   atatctggca ataaaaataa taaatatata ttttttaata aatgaatcaa ccacttaatt 10080
   tttctgtaaa tatctgtaac ttctcttctg tctttccaaa aacactcata agtactgtga 10140
   atgagatgaa aaagagtgaa gtaggatata ggctgttagc agaaaacatc tgaatggctg 10200
   gcagtgaaac attaacttga aatgtaagat taatgagtaa tagtaaattt taaccttggc 10260
   catatgataa aatgttcatt aatatttttc tagaatacag ggctttttgt ttttgccatg 10320
   aggtttgcag gatcttggtt ccctgaccag ggatcaaacc tgcacaccag ggatcaaacc 10380
   tgcactcccc tggaagcatg gagtcttgga catttgtatt atacactatc tttggttcct 10440
   tttaaaggga agtaatttta cttaaataag aaaatagatt gacaagtaat acgctgtttc 10500
   ctcatcttcc cattcacagg aatcgcggat cctcgacgcc atgaaggtcc tcatccttgc 10560
   ctgtctggtg gctctggcca ttgctcgagg atccggaccc ttccctattc ttgtaagtct 10620
   aaatttacta actgtgctgt ttaacttctg atgtttgtat gatatttgag taattaagag 10680
   ccctacaaaa aaatcaataa tgaatggttc caaaataagc atagctgaga ttaatgattc 10740
   tcagcattag ttataaatag aataagctgg aaaaccttca cctcccctcc accaccagat 10800
   ctcaatgtct aggcttaccc atggagattc tgattaactg ttctttctat gtagaagaaa 10860
   cttattggga agaaataata taatggacta tgatttaatt ggtctgttga gaatttagat 10920
   gaaggggatt aagttacaat aaagccagaa tttaacttga taatctcatt tggctaagaa 10980
   taacaaacct aagaaggttt gctattttct acaattttga agttttcctt atgcacaatt 11040
   atttcaccac atgactcatt tcacatcttg tttttgatat atgagcatat gagggcaaaa 11100
   tactgaagat gcttatttca atactcaggg aaaattttct tgccaaaagg caagaattgt 11160
   ataattcatt cacttatttt atttttttta atttttaagg tctaagagga tttcaaagtg 11220
   aatgccccct cctcactttt ggtaagcttt aggagattgg aggcagactg atcattttta 11280
   tagttaatat cttttacatt tcatcttcct ggataagccc caatagtagc aatttctatc 11340
   agtataccag cataaagatt agttttaaat ttattttcag tgattgactg ttatttactg 11400
   acctgaaatt atgtatctgt tatatttcaa ataatgcaaa actgtatata tatggtgttg 11460
   acagatttga ttggttttct ttcaattgcc tatatcctta ttattgattg taatcattta 11520
   tagaaaaaac aaaataattt cttatacttt tatgtaaacc tgttagagct tattttaaag 11580
   atcaactgca ttcacatttc taatctagtc attatgagct tcaattgttt tatctcactt 11640
   aaaatttata tattgtcttt taattcatga gtcaaaatac aatctcacag tccagatatg 11700
   ggacttaaaa ggggaatagc atatagtttt gatattctta aagatataca tctttttgtg 11760
   atcatgattc agcagacatt ttaataaaac aattccaagt gagccgacac ttggtcctag 11820
   aggaattttt ataaccttaa gataaggcac agcatggtgt ttttgtaata agatttcttt 11880
   tatgaaaaag tcacaccaaa attggaaatg gggtgagatg aagagttata acatataact 11940
   aaatggacat ttgttctcta ttccacagaa ttgactgcga ctggaaatat ggcaactttt 12000
   caatccttgc atcatgctac taagataatt tttaaatgag tatacatgga acaaaaaatg 12060
   aaactttatt cctttattta tattatgctt tttcatctta atttgaattt gagtcataaa 12120
   ccatatactt tcaaaatgtt aattcaacat tagcataaaa gttcaatttt aacttggaaa 12180
   tatcatgaac atatcaaatt atgtataaaa ataatttctg gaattgtgat tattatttct 12240
   ttaagaatct atttcctaac cagtcatttc aataaattaa cccttaggca tatttaagtt 12300
   ttcttgtctt tattatattt ttaaaaatga aattggtctc tttattgtta acttaaattt 12360
   atctttgatg ttaaaaatag ctgtggaaaa ttaaaattga atagaattct ttgaattgag 12420
   ttccaaagga tatcaaaaag tgagggaaaa gatagggtga gcctatgctg catatgtcct 12480
   tagaaagtct tggtttatac ctgttaccta agttaaacaa ttatacttgt tcctttcact 12540
   ctcgaaagta cccagcattg gatgttaaat tttatagtca tcctagacaa aaaaaaaaaa 12600
   aaaaacaaac aaccctcaaa tgtgatatct gaatcacagc tctacagtgt ggtagctaag 12660
   tggtgctgtg taagttagtc tccaagagat tccatttcta catttataaa cagtcaattt 12720
   aaggtgtttt attgaagttt taatgtgaaa agtgcactat atggtgcatg ataggagttc 12780
   ctggttgaat ctcatttctg acatcactga caccagtgca gcaaggacta gagcggccgc 12840
   atgcataagc ttgagtattc tatagtgtca cctaaatagc ttggcgtaat catggtcata 12900
   gctgtttcct gtgtgaaatt gttatccgct cacaattcca cacaacatac gagccggaag 12960
   cataaagtgt aaagcctggg gtgcctaatg agtgagctaa ctcacattaa ttgcgttgcg 13020
   ctcactgccc gctttccagt cgggaaacct gtcgtgccag ctgcattaat gaatcggcca 13080
   acgcgcgggg agaggcggtt tgcgtattgg gcgctcttcc gcttcctcgc tcactgactc 13140
   gctgcgctcg gtcgttcggc tgcggcgagc ggtatcagct cactcaaagg cggtaatacg 13200
   gttatccaca gaatcagggg ataacgcagg aaagaacatg tgagcaaaag gccagcaaaa 13260
   ggccaggaac cgtaaaaagg ccgcgttgct ggcgtttttc cataggctcc gcccccctga 13320
   cgagcatcac aaaaatcgac gctcaagtca gaggtggcga aacccgacag gactataaag 13380
   ataccaggcg tttccccctg gaagctccct cgtgcgctct cctgttccga ccctgccgct 13440
   taccggatac ctgtccgcct ttctcccttc gggaagcgtg gcgctttctc atagctcacg 13500
   ctgtaggtat ctcagttcgg tgtaggtcgt tcgctccaag ctgggctgtg tgcacgaacc 13560
   ccccgttcag cccgaccgct gcgccttatc cggtaactat cgtcttgagt ccaacccggt 13620
   aagacacgac ttatcgccac tggcagcagc cactggtaac aggattagca gagcgaggta 13680
   tgtaggcggt gctacagagt tcttgaagtg gtggcctaac tacggctaca ctagaagaac 13740
   agtatttggt atctgcgctc tgctgaagcc agttaccttc ggaaaaagag ttggtagctc 13800
   ttgatccggc aaacaaacca ccgctggtag cggtggtttt tttgtttgca agcagcagat 13860
   tacgcgcaga aaaaaaggat ctcaagaaga tcctttgatc ttttctacgg ggtctgacgc 13920
   tcagtggaac gaaaactcac gttaagggat tttggtcatg agattatcaa aaaggatctt 13980
   cacctagatc cttttaaatt aaaaatgaag ttttaaatca atctaaagta tatatgagta 14040
   aacttggtct gacagttacc aatgcttaat cagtgaggca cctatctcag cgatctgtct 14100
   atttcgttca tccatagttg cctgactccc cgtcgtgtag ataactacga tacgggaggg 14160
   cttaccatct ggccccagtg ctgcaatgat accgcgagac ccacgctcac cggctccaga 14220
   tttatcagca ataaaccagc cagccggaag ggccgagcgc agaagtggtc ctgcaacttt 14280
   atccgcctcc atccagtcta ttaattgttg ccgggaagct agagtaagta gttcgccagt 14340
   taatagtttg cgcaacgttg ttgccattgc tacaggcatc gtggtgtcac gctcgtcgtt 14400
   tggtatggct tcattcagct ccggttccca acgatcaagg cgagttacat gatcccccat 14460
   gttgtgcaaa aaagcggtta gctccttcgg tcctccgatc gttgtcagaa gtaagttggc 14520
   cgcagtgtta tcactcatgg ttatggcagc actgcataat tctcttactg tcatgccatc 14580
   cgtaagatgc ttttctgtga ctggtgagta ctcaaccaag tcattctgag aatagtgtat 14640
   gcggcgaccg agttgctctt gcccggcgtc aatacgggat aataccgcgc cacatagcag 14700
   aactttaaaa gtgctcatca ttggaaaacg ttcttcgggg cgaaaactct caaggatctt 14760
   accgctgttg agatccagtt cgatgtaacc cactcgtgca cccaactgat cttcagcatc 14820
   ttttactttc accagcgttt ctgggtgagc aaaaacagga aggcaaaatg ccgcaaaaaa 14880
   gggaataagg gcgacacgga aatgttgaat actcatactc ttcctttttc aatattattg 14940
   aagcatttat cagggttatt gtctcatgag cggatacata tttgaatgta tttagaaaaa 15000
   taaacaaata ggggttccgc gcacatttcc ccgaaaagtg ccacctgacg tctaagaaac 15060
   cattattatc atgacattaa cctataaaaa taggcgtatc acgaggccct ttcgtctcgc 15120
   gcgtttcggt gatgacggtg aaaacctctg acacatgcag ctcccggaga cggtcacagc 15180
   ttgtctgtaa gcggatgccg ggagcagaca agcccgtcag ggcgcgtcag cgggtgttgg 15240
   cgggtgtcgg ggctggctta actatgcggc atcagagcag attgtactga gagtgcacca 15300
   tatgcggtgt gaaataccgc acagatgcgt aaggagaaaa taccgcatca ggacgcgccc 15360
   tgtagcggcg cattaagcgc ggcgggtgtg gtggttacgc gcagcgtgac cgctacactt 15420
   gccagcgccc tagcgcccgc tcctttcgct ttcttccctt cctttctcgc cacgttcgcc 15480
   ggctttcccc gtcaagctct aaatcggggg ctccctttag ggttccgatt tagtgcttta 15540
   cggcacctcg accccaaaaa acttgattag ggtgatggtt cacgtagtgg gccatcgccc 15600
   tgatagacgg tttttcgccc tttgacgttg gagtccacgt tctttaatag tggactcttg 15660
   ttccaaactg gaacaacact caaccctatc tcggtctatt cttttgattt ataagggatt 15720
   ttgccgattt cggcctattg gttaaaaaat gagctgattt aacaaaaatt taacgcgaat 15780
   tttaacaaaa tattaacgct tacaatttcc attcgccatt caggctgcgc aactgttggg 15840
   aagggcgatc ggtgcgggcc tcttcgctat tacgccagct ggcgaaaggg ggatgtgctg 15900
   caaggcgatt aagttgggta acgccagggt tttcccagtc acgacgttgt aaaacgacgg 15960
   ccagtgaatt gtaatacgac tcactata 15988
<210> 3
   <211> 606
   <212> DNA
   <213> Artificial Sequence
<220>
   <223> hEPO cDNA
<400> 3
   atgaaggtcc tcatccttgc ctgtctggtg gctctggcca ttgctcgagg gggttctcac      60
   catcaccatc accatccatg ggattacaag gacgacgatg acaaggcccc accacgcctc     120
   atctgtgaca gccgagtcct ggagaggtac ctcttggagg ccaaggaggc cgagaatatc     180
   acgacgggct gtgctgaaca ctgcagcttg aatgagaata tcactgtccc agacaccaaa     240
   gttaatttct atgcctggaa gaggatggag gtcgggcagc aggccgtaga agtctggcag     300
   ggcctggccc tgctgtcgga agctgtcctg cggggccagg ccctgttggt caactcttcc     360
   cagccgtggg agcccctgca gctgcatgtg gataaagccg tcagtggcct tcgcagcctc     420
   accactctgc ttcgggctct gggagcccag aaggaagcca tctcccctcc agatgcggcc     480
   tcagctgctc cactccgaac aatcactgct gacactttcc gcaaactctt ccgagtctac     540
   tccaatttcc tccggggaaa gctgaagctg tacacagggg aggcctgcag gacaggggac     600
   agatga                                                                606
<210> 4
   <211> 201
   <212> PRT
   <213> Homo sapiens
<220>
   <223> Erythropoietin protein
<400> 4
   Met Lys Val Leu Ile Leu Ala Cys Leu Val Ala Leu Ala Ile Ala Arg
   1               5                   10                  15
   Gly Gly Ser His His His His His His Pro Trp Asp Tyr Lys Asp Asp
               20                  25                  30
   Asp Asp Lys Ala Pro Pro Arg Leu Ile Cys Asp Ser Arg Val Leu Glu
           35                  40                  45
   Arg Tyr Leu Leu Glu Ala Lys Glu Ala Glu Asn Ile Thr Thr Gly Cys
       50                  55                  60
   Ala Glu His Cys Ser Leu Asn Glu Asn Ile Thr Val Pro Asp Thr Lys
   65                  70                  75                  80
   Val Asn Phe Tyr Ala Trp Lys Arg Met Glu Val Gly Gln Gln Ala Val
                   85                  90                  95
   Glu Val Trp Gln Gly Leu Ala Leu Leu Ser Glu Ala Val Leu Arg Gly
               100                 105                 110
   Gln Ala Leu Leu Val Asn Ser Ser Gln Pro Trp Glu Pro Leu Gln Leu
           115                 120                 125
   His Val Asp Lys Ala Val Ser Gly Leu Arg Ser Leu Thr Thr Leu Leu
       130                 135                 140
   Arg Ala Leu Gly Ala Gln Lys Glu Ala Ile Ser Pro Pro Asp Ala Ala
   145                 150                 155                 160
   Ser Ala Ala Pro Leu Arg Thr Ile Thr Ala Asp Thr Phe Arg Lys Leu
                   165                 170                 175
   Phe Arg Val Tyr Ser Asn Phe Leu Arg Gly Lys Leu Lys Leu Tyr Thr
               180                 185                 190
   Gly Glu Ala Cys Arg Thr Gly Asp Arg
           195                 200
<210> 5
   <211> 636
   <212> DNA
   <213> Artificial Sequence
<220>
   <223> Growth hormone open reading frame (orf)
<400> 5
   atgaaggtcc tcatccttgc ctgtctggtg gctctggcca ttgctcgagg gggttctcac      60
   catcaccatc accatccatg ggattacaag gacgacgatg acaagttccc aaccattccc     120
   ttatccaggc tttttgacaa cgctatgctc cgcgcccatc gtctgcacca gctggccttt     180
   gacacctacc aggagtttaa cccccagacc tccctctgtt tctcagagtc tattccgaca     240
   ccctccaaca gggaggaaac acaacagaaa tccaacctag agctgctccg catctccctg     300
   ctgctcatcc agtcgtggct ggagcccgtg cagttcctca ggagtgtctt cgccaacagc     360
   ctggtgtacg gcgcctctga cagcaacgtc tatgacctcc taaaggacct agaggaaggc     420
   atccaaacgc tgatggggag gctggaagat ggcagccccc ggactgggca gatcttcaag     480
   cagacctaca gcaagttcga cacaaactca cacaacgatg acgcactact caagaactac     540
   gggctgctct actgcttcag gaaggacatg gacaaggtcg agacattcct gcgcatcgtg     600
   cagtgccgct ctgtggaggg cagctgtggc ttctag                               636
<210> 6
   <211> 211
   <212> PRT
   <213> Homo sapiens
<220>
   <223> Growth hormone protein
<400> 6
   Met Lys Val Leu Ile Leu Ala Cys Leu Val Ala Leu Ala Ile Ala Arg
   1               5                   10                  15
   Gly Gly Ser His His His His His His Pro Trp Asp Tyr Lys Asp Asp
               20                  25                  30
   Asp Asp Lys Phe Pro Thr Ile Pro Leu Ser Arg Leu Phe Asp Asn Ala
           35                  40                  45
   Met Leu Arg Ala His Arg Leu His Gln Leu Ala Phe Asp Thr Tyr Gln
       50                  55                  60
   Glu Phe Asn Pro Gln Thr Ser Leu Cys Phe Ser Glu Ser Ile Pro Thr
   65                  70                  75                  80
   Pro Ser Asn Arg Glu Glu Thr Gln Gln Lys Ser Asn Leu Glu Leu Leu
                   85                  90                  95
   Arg Ile Ser Leu Leu Leu Ile Gln Ser Trp Leu Glu Pro Val Gln Phe
               100                 105                 110
   Leu Arg Ser Val Phe Ala Asn Ser Leu Val Tyr Gly Ala Ser Asp Ser
           115                 120                 125
   Asn Val Tyr Asp Leu Leu Lys Asp Leu Glu Glu Gly Ile Gln Thr Leu
       130                 135                 140
   Met Gly Arg Leu Glu Asp Gly Ser Pro Arg Thr Gly Gln Ile Phe Lys
   145                 150                 155                 160
   Gln Thr Tyr Ser Lys Phe Asp Thr Asn Ser His Asn Asp Asp Ala Leu
                   165                 170                 175
   Leu Lys Asn Tyr Gly Leu Leu Tyr Cys Phe Arg Lys Asp Met Asp Lys
               180                 185                 190
   Val Glu Thr Phe Leu Arg Ile Val Gln Cys Arg Ser Val Glu Gly Ser
           195                 200                 205
   Cys Gly Phe
       210
<210> 7
   <211> 366
   <212> DNA
   <213> Artificial Sequence
<220>
   <223> Insuline open reading frame (orf)
<400> 7
   atgaaggtcc tcatccttgc ctgtctggtg gctctggcca ttgctcgagg gggttctcac      60
   catcaccatc accatccatg ggattacaag gacgacgatg acaagtttgt gaaccaacac     120
   ctgtgcggct cacacctggt ggaagctctc tacctagtgt gcggggaacg aggcttcttc     180
   tacacaccca agacccgccg ggaggcagag gacctgcagg tggggcaggt ggagctgggc     240
   gggggccctg gtgcaggcag cctgcagccc ttggccctgg aggggtccct gcagaagcgt     300
   ggcattgtgg aacaatgctg taccagcatc tgctccctct accagctgga gaactactgc     360
   aactag                                                                366
<210> 8
   <211> 121
   <212> PRT
   <213> Homo sapiens
<220>
   <223> Insuline protein
<400> 8
   Met Lys Val Leu Ile Leu Ala Cys Leu Val Ala Leu Ala Ile Ala Arg
   1               5                   10                  15
   Gly Gly Ser His His His His His His Pro Trp Asp Tyr Lys Asp Asp
               20                  25                  30
   Asp Asp Lys Phe Val Asn Gln His Leu Cys Gly Ser His Leu Val Glu
           35                  40                  45
   Ala Leu Tyr Leu Val Cys Gly Glu Arg Gly Phe Phe Tyr Thr Pro Lys
       50                  55                  60
   Thr Arg Arg Glu Ala Glu Asp Leu Gln Val Gly Gln Val Glu Leu Gly
   65                  70                  75                  80
   Gly Gly Pro Gly Ala Gly Ser Leu Gln Pro Leu Ala Leu Glu Gly Ser
                   85                  90                  95
   Leu Gln Lys Arg Gly Ile Val Glu Gln Cys Cys Thr Ser Ile Cys Ser
               100                 105                 110
   Leu Tyr Gln Leu Glu Asn Tyr Cys Asn
           115                 120

## Description

### Field of the invention

This invention relates to a method for generating a non-human transgenic animal by genetic engineering, in particular, the invention relates to a method for producing transgenic cloned bovine embryos using transgenic cell lines.

### Background of the Invention

Various cloning technologies and methods for the preparation of non-human transgenic cloned embryos have already been developed earlier. In patent document EE 03074 B1 of 15 April 1996, a recombinant DNA technique has been described. More specifically, the said document relates to new recombinant eucaryotic cells which are transformed with SSO genes. Eucaryotic cells that have been transformed with several copies of SSO genes or that overexpress SSO protein in any other way exhibit an increased capacity to produce secreted foreign or endogenous proteins. Further, these new recombinant cells, when transformed with genes expressing suitable hydrolytic enzymes, can utilize appropriate macromolecular compounds more efficiently, which results in increased cell mass production and/or more versatile utilization of the compounds in relevant biotechnical applications. In European patent EP 1 217072 B1 of 22 July 2009, the preparation of expression vectors used for therapeutic protein production in mammary glands of non-human transgenic animals is described. These are mainly vectors constructed by modifying nucleotide sequences.
In European patent EP 0546091 of 10 March 1992, generation of a transgenic animal and homologous recombination of mammalian cells are described.

Among the drawbacks of earlier methods for generation of non-human transgenic animals, especially the complexity of the cloning procedure and low efficiency should be noted.

Together with chemical synthesis and recombinant production, the technique of transgenic production is one of the three main methods for the production of therapeutic proteins. Each of the said technologies has certain advantages and disadvantages.

One of the most potent future methods for large-scale production of long-chain peptides is expected to be pharming. The term "pharming" refers to creating therapeutic proteins through non-human transgenic (TG) animals. In pharming, a non-human animal is used as a bioreactor in which proteins or peptides foreign to its body (including therapeutic proteins and peptides) are synthesised in the milk, blood, or urine of the non-human animal. Pharming is an extremely attractive method of producing peptides, since the milk of farm non-human animals contains dozens of grams of protein per one litre of milk and it is possible to maintain current operating costs at a comparatively low level. Further, the use of a transgenic farm non-human animal as a so-called bioreactor is relatively unlimited area (milk comprises six major proteins that occur naturally in most mammals). As regards the costs, production of long chain peptides using this method is remarkably cheaper than recombinant production, and despite the fact that in the case of short chains, chemical synthesis remains the cheapest method, pharming becomes the most cost-effective way of production if the chains contain more than 15 amino acids.

Concerning transgenic production of proteins, it should be noted that in most cases, pharming is based on manipulating the content of milk in non-human transgenic animals. As an advantage of the disclosed method, also a remarkably large quantity of the desired product and non-invasiveness of the method (milking of cows) are noteworthy.

### Summary of the invention

The aim of this invention is to provide a method for obtaining biopharmaceuticals, such as growth hormone, human insulin or human erythropoietin from transgenic bovine animals, wherein the cDNA sequences of human growth hormone, human insulin or human erythropoietin are inserted into the genome of a transgenic bovine, using a pMilk expression vector.
In order to generate a transgenic bovine necessary for the application of the method, nuclear transfer, DNA nuclear microinjection, DNA transfer with non-human embryonic stem cells (including gene targeting technology), DNA vector transfer with viral vectors, DNA transfer with penetrating peptides, sperm-mediated DNA vector transfer, *in vitro* and *in vivo* DNA transfer via DNA transfections are used. For the expression of proteins and peptides in milk, a pMILK expression vector is used.
In order to achieve the purposes of the method, non-human transgenic cell lines are used to generate bovine transgenic cloned embryos. For the production of non-human transgenic cell lines, primary cell lines grown from bovine embryonic skin tissue, primary fibroblasts are used as donor cells. Into these cells of non-human primary embryonic fibroblast cell line, a recombinant plasmid is inserted into which, under the influence of β-casein promoter, cDNA sequences encoding human insulin (hINS), growth hormone (hGH), or erythropoietin (hEPO) have been cloned. Resulting plasmid vectors (hEPO, hGM, hINS) are aligned with restrictases Not I and Eco136II, the plasmid is electroporated into the cells and then a neomycin (G418) resistance test is performed to select non-human transgenic cells.

Ova necessary for cloning are collected by aspiration under vacuum from ovaries obtained from an abattoir which are washed beforehand with 0.9% NaCl solution. Selected ova are matured in TCM-199 medium for 16 to 17 hours. After that, cumulus cells are removed from matured ova with hyaluronidase and using a micromanipulator, the polar body and the nucleus are removed from the ova and the prepared non-human transgenic fibroblast cell is injected into the perivitelline space. For the donor cell and the ovum to be fused, they are subjected to an electrical field (electrofusion for the nuclear transfer) and after that, to chemical activation. Non-human transgenic cloned embryos are cultivated until they develop into the non-human blastocyst stage and then they are implanted into prepared recipients.

### Brief Description of the Drawings

The method provided herein for the use of non-human transgenic cell lines is described in detail below with reference to the figures which illustrate the embodiments of the invention, wherein:
Figure 1 depicts the described DNA expression base vector pMILK (SEQ No. 2) for expressing different biomolecules in cow's milk, whereas the length of the base vector is 15,246 base-pairs;
Figure 2 is a diagram showing recombinant DNA together with the domains of the secreted protein, as illustrated by hINS;
Figure 3 depicts the described DNA expression base vector for expressing different biomolecules in cow's milk; the length of the shown base vector is 15,998 base-pairs and the only difference in relation to the pMILK vector is the site of the Neo cassette. Using this vector makes it possible to remove the Neo cassette and obtain non-human transgenic systems without the antibiotic resistance gene;
Figure 4 is a diagram showing recombinant DNA together with the domains of the secreted protein, as illustrated by hINS, wherein the base vector of Figure 3 serves as the basis.

### Detailed Description of the Invention

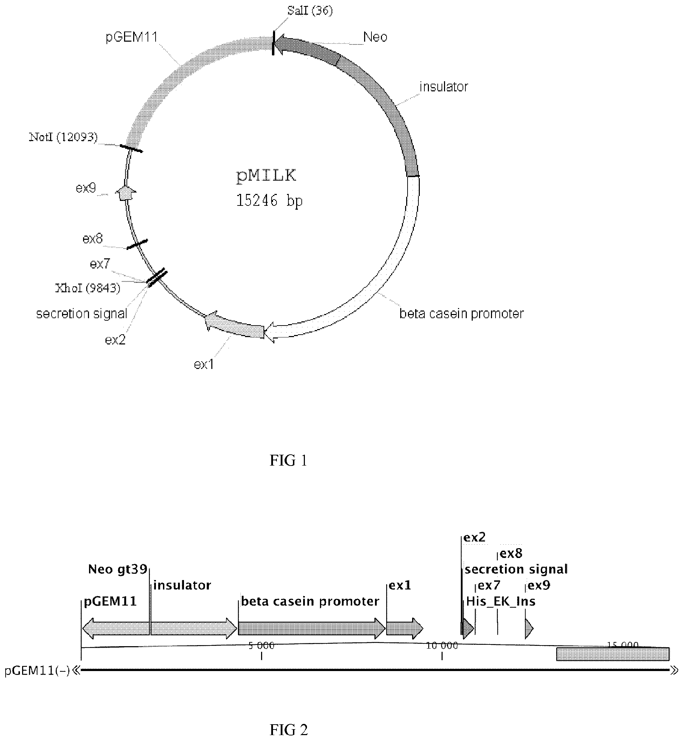

In order to generate bovine transgenic cloned embryos, non-human transgenic cell lines are used. As donor cells for producing non-human transgenic cell lines, primary cell lines grown from the skin tissue of two-month-old non-human embryos obtained from Valga Abattoir were used. Two primary bovine embryonic fibroblast cell lines were selected which have so far given the highest non-human blastocyst recovery in cloning experiments. The system used for preparing non-human transgenic cell lines was Lonza Amaxa Basic Nucleofector Kit for Primary Mammalian Fibroblast, which was used according to the manufactures protocol with slight in-house modifications. Into the cells, a pMILK plasmid (Fig. 1) was inserted into which, after the β-casein promoter, cDNA sequences encoding human insulin (hINS), growth hormone (hGH), or erythropoietin (hEPO) had been cloned. The resulting plasmid vectors (hEPO, hGH, hINS) were aligned for electroporation with restrictases Not I and Eco136II. To select non-human transgenic cells, a neomycin (G418) resistance test with the concentration of 800 ng/ml was used. Non-human transgenic cell lines were grown in bulk which was then divided between vials and frozen for storing at -80 °C. PCR tests were performed to check the non-human transgenic cell lines. For generation of cloned non-human embryos, a corresponding non-human transgenic cell line is thawed and cultivated to confluence.

The length of the base vector in Figure 1 of the pMILK plasmid integrated into the cells is 15,246 base-pairs. The vector contains a β-casein promoter sequence, milk secretion signal, a G418 resistance cassette, an insulator sequence, and a multiple cloning site.

The vector comprises of the following components:
(a) a chicken beta-globin insulator sequence (in double repeat, obtained from Invitrogen pBC1 vector);
(b) a goat beta-casein promoter sequence (obtained from Invitrogen pBC1 vector);
(c) a goat beta-casein gene exon 1, intron 1, exon 2, exon 7, intron 7, exon 8, intron 8, exon 9, whereas exons 1 and 2 are the non-translated 5-UTR region, exon 7 to exon 9 is the 3' non-translated region, and integrated inserts are surrounded by exon 2 and exon 7 (obtained from Invitrogen pBC1 vector);
(e) all inserts have been integrated into the pGEM-11Zf (+) cloning vector (Promega);
(f) in the cloning vector pGEM-11Zf (+), a low copy ori of the bacter is replaced by a high copy ori;
(g) into the vector, a region encoding goat milk secretion is inserted in the GOI 5' terminus (MKVLILACLVALAIA, **SEQ No. 1**). The signal is intended for secreting milk from the cells of the expressed proteins. GOI is a cloned XhoI site;
(h) a NEO mycin (G418) resistance cassette or selection marker (from neo plasmid pGT-N39, New England BioLabs.) is cloned into the vector. In relation to the casein promoter, the cassette is located in the opposite direction and has been separated from it by an insulator sequence;
(i) the bacterial sequence is removed with Sall/NotI.
Figure 2 is the diagram showing recombinant DNA together with the domains of the secreted protein, as illustrated by hINS. hEPO (SEQ No. 3 or SEQ No. 4), hGH (SEQ No. 5 or SEQ No. 6) or hINS (SEQ No. 7 or SEQ No. 8) are translated together with the secretion signal of the goat beta-casein with 6xHis tag domain and the enterokinase sequence. The signal sequence of beta-casein ensures the transportation of the protein into milk, 6xHis-tag makes it possible to purify it from milk and the sequence of enterokinase provides an opportunity to separate the signal sequence and his-tag sequences from hEPO, hGH or hINS. This results in a corresponding hormone lacking his-tag and the domain of the secretion signal.

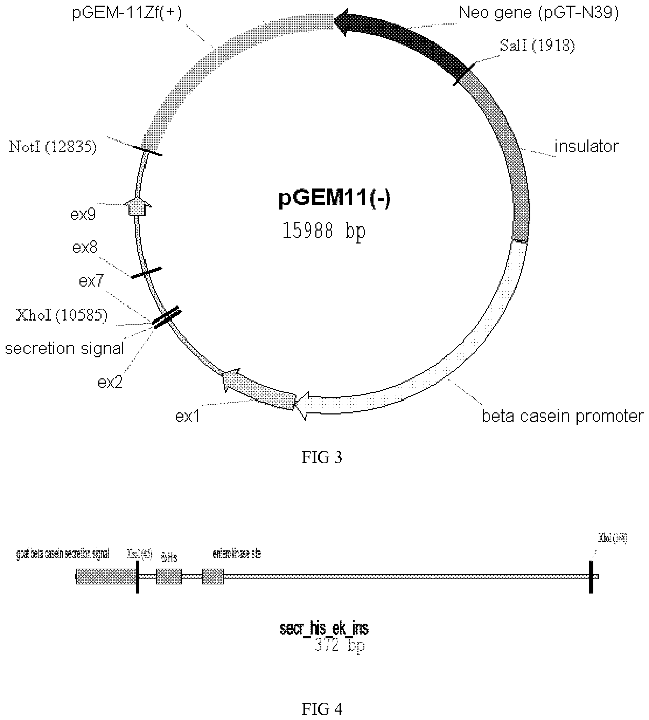

The base vector of the pMILK plasmid inserted into the cell and shown in Figure 3 is of the length of 15,246 base-pairs. The vector contains a β-casein promoter sequence, a milk secretion signal, a G418 resistance cassette, an insulator sequence, and a multiple cloning site.

The vector comprises of the following components:
(a) a chicken beta-globin insulator sequence (in double repeat, obtained from Invitrogen pBC1 vector);
(b) a goat beta-casein promoter sequence (obtained from Invitrogen pBC1 vector);
(c) a goat beta-casein gene exon 1, intron 1, exon 2, exon 7, intron 7, exon 8, intron 8, exon 9, whereas exons 1 and 2 are the non-translated 5-UTR region, exon 7 to exon 9 is the 3' non-translated region, and integrated inserts are surrounded by exon 2 and exon 7 (obtained from Invitrogen pBC1 vector);
(e) all inserts have been integrated into the pGEM-11Zf (+) cloning vector (Promega);
(f) in the cloning vector pGEM-11Zf (+), a low copy ori of the bacter is replaced by a high copy ori;
(g) into the vector, a region encoding goat milk secretion is inserted in the GOI 5' terminus (MKVLILACLVALAIA, **SEQ No. 1).** The signal is intended for secreting milk from the cells of expressed proteins. GOI is a cloned XhoI site;
(h) a NEO mycin (G418) resistance cassette or selection marker (from neo plasmid pGT-N39, New England BioLabs.) is cloned into the vector. In relation to the casein promoter, the cassette is located in the opposite direction and has been separated from it by an insulator sequence;
(i) the bacterial sequence and the NEO mycine (G418) resistance cassette are removed with Sall/NotI.
Figure 4 is the diagram showing recombinant DNA together with the domains of the secreted protein as illustrated by hINS and the base vector of Figure 3 provides a basis. It is different from Figure 2 in that this vector does not contain a NEO mycine resistance cassette any longer. hEPO (SEQ No. 3 or SEQ No. 4), hGH (SEQ No. 5 or SEQ No. 6) or hINS (SEQ No. 7 or SEQ No. 8) are translated together with the secretion signal of the goat beta-casein with 6 x His-tag domain and the enterokinase sequence. The signal sequence of beta-casein ensures the transportation of the protein into milk, 6xHis-tag makes it possible to purify it from milk and the sequence of enterokinase provides an opportunity to separate the signal sequence and his-tag sequences from hEPO, hGH or hINS. This results in a corresponding hormone lacking his-tag and the domain of the secretion signal.
Ova necessary for cloning are collected by aspiration under vacuum from ovaries obtained from an abattoir and washed beforehand with 0.9% NaCl solution. Selected ova are matured in TCM-199 medium for 16 to 17 hours. Then, the cumulus cells are removed from the matured ova with hyaluronidase. Using a micromanipulator, the polar bodies and the nuclei are removed from the ova and a prepared non-human transgenic fibroblast cell is injected into the perivitelline space. For the donor cell and the ovum to be fused, they are subjected to an electrical field and after that, to chemical activation. Non-human transgenic cloned embryos are cultivated until they develop into the non-human blastocyst stage. Finally, they are implanted into the prepared recipients.
The method according to the invention includes steps in which for the preparation of non-human transgenic cell lines:
a. bovine fibroblast cell lines were first grown to confluence in DMEM medium supplemented with 10% fetal bovine serum and 1X MEM,
b. the cells were trypsinized and counted (1x106 cells accounted for one poration),
c. after that the cells were suspended in 100 µl Nucleofector solution and 5-30 µg of aligned hEPO (SEQ No. 3 or SEQ No. 4), hGH (SEQ No. 5 or SEQ No. 6) or hINS (SEQ No. 7 or SEQ No. 8) vector was added,
d. the program U-023 was used for poration,
e. after poration, cells were cultivated in DMEM medium supplemented with 10% fetal bovine serum and 1X MEM for 2 weeks, then
f. the cells were frozen in DMEM medium supplemented with 20% fetal bovine serum and 10% DMSO.
As the next step, cells were prepared for cloning by
g. first thawing the cells in 10 ml of DMEM medium supplemented with 10% fetal bovine serum and 1X MEM,
h. then suspending the cells in a 24-well plate in DMEM medium supplemented with 10% fetal bovine serum and 1X MEM,
i. replacing the medium 48 h later and
j. trypnisizing the cells 96 h later, centrifuging and suspending them in 0.4% solution of BSA/PBS.
Cloning included steps wherein
a. the ovaries were transported from the abattoir to the laboratory in a vacuum flask at 39 °C in a solution of 0.9% NaCl,
b. the ovaries were washed three times in a total of 1 litre of 0.9% NaCl solution,
c. the ova were aspirated from the follicles with the diameter of 2-7 mm using a vacuum pump, 9 ml evacuated tubes and 18 G needles,
d. the ova were washed 2 times with 3 ml of Hepes-TCM solution (Hepes-TCM-199, sodium carbonate 2.2 mg/ml, PVA (polyvinylalcohol) 0.5 mg/ml, L-glutamine 0.3 mM, gentamycine 50 µg/ml),
e. the ova were incubated for 16-17 hours in TCM-199 maturation solution (TCM-199 4.5 ml, ECS (fetal bovine serum, tested on non-human embryonic stem cells) 10%, sodium pyruvate 0.5 mM, L-glutamine 1 mM, gentamycine 50 µg/ml, EGF, Suigonan PG600),
f. follicular cells were isolated from the mature ova with 0.5 mg/ml hyaluronidase Hepes-TCM-199 solution,
g. the ova were enucleated with micromanipulator in 0.4% solution of BSA/PBS (fetal bovine serum albumin/phosphate-buffered saline),
h. using the micromanipulator, fibroblast cell lines (non-human transgenic cell lines hEPO (SEQ No. 3 or SEQ No. 4), hGH (SEQ No. 5 or SEQ No. 6) or hINS (SEQ No. 7 or SEQ No. 8) were injected into the ova,
i. electrofusion was performed using the following parameters: 70V 25µsec 2p, 65V 25µsec 1p (fusion apparatus Eppendorf multiporator, fusion chamber 0.5 mm "gap"),
j. after the fusion, cloned non-human embryos were incubated for 3 hours in an incubator (at 39 °C and 5.5% CO₂) in Ham's F12 solution containing 10% ECS and sodium pyruvate,
k. activation: 4 minutes incubation in 5 µg/ml calcium ionophore SOFaaci medium with a solution containing 5% ECS, 1X BMEM (essential amino acids), IX MEM (non-essential amino acids), 0.3 g/L sodium pyruvate. After activation, the non-human embryos were washed three times in 0.5 ml SOFaaci medium supplemented with 10% ECS, IX essential amino acids, 1X non-essential amino acids, 0.3 g/L sodium pyruvate,
l. the cloned non-human embryos were incubated for 5 hours in an incubator (at 39 °C and 5.5% CO₂) in 500 µl of 2 mM 6-DMAP (6-dimethylamino-pyridine) SOFaaci solution supplemented with 10% ECS, 1X essential amino acids, 1X non-essential amino acids, 0.3 g/L sodium pyruvate,
m. after treating with 6-DMAP, the non-human embryos were washed three times in 0.5 ml SOFaaci medium supplemented with 5% fetal bovine serum, 1X essential amino acids, 1X non-essential amino acids, 0.3 g/L sodium pyruvate,
n. cloned non-human embryos were cultivated in a multiple gas incubator (at 39 °C, 5.5% CO₂, 5% O₂) for 7 days in SOFaaci medium supplemented with 5% fetal bovine serum, 1X essential amino acids, 1X non-essential amino acids, 0.3 g/L sodium pyruvate,
o. the zona pellucida of the cloned non-human embryo was opened on the fifth day with a micromanipulator in 0.4% solution of BSA/PBS. After that, standard incubation in SOFaaci medium supplemented with 5% fetal bovine serum, 1X essential amino acids, 1X non-essential amino acids, and 0.3 g/L sodium pyruvate was performed.
As an alternative solution, also the use of a cell line originating from skin cells of a cloned transgenic calf containing human growth hormone of a healthy person was tested in cloning. The said primary transgenic cell line contained the gene of human growth hormone already integrated in the genome. PCR and sequencing was performed to test the cell line and verify if the cell line was transgenic or not. Compared with the earlier transgenic cell lines, the obtained cell line was more suitable for cloning, because it originated from a living transgenic entity, which allows for a conclusion that upon cloning, it is capable of giving living offspring. In addition, as the cell line is already transgenic, there is no need to perform any additional genetic manipulations with the primary cell line which might be time-consuming and may affect the viability of cells as well as non-human embryos obtained when using them. In earlier stages, non-human transgenic cell lines were used in cloning which were prepared by electroporation and selection with antibiotics, which does not ensure a uniform quality of cloning, because the obtained cell population is not homogeneous, but extremely heterogeneous, i.e. each cell is essentially different as the integration of the transgene into the genome is achieved by non-directed recombination. The cell line grown from a sample of skin tissue of a transgenic calf is completely homogeneous, which should ensure a more uniform quality of cloning and also a greater likelihood of the birth of a viable transgenic calf after implantation of cloned non-human embryos.
The new non-human transgenic cell line contained the cDNA sequence with human growth hormone under the control of a β-caseine promoter, which should ensure the synthesis of growth hormone only in the cells of the mammary gland. A number of cloning tests (15) were performed with the said cell line, wherein 29 out of 339 non-human transgenic embryos or 8.5% developed into the non-human blastocyst stage. The efficiency of earlier cell lines with growth hormone which have been used for cloning has been almost the same. For example, in experiments performed with a cow containing 2 GH cell lines, the cloning efficiency was 13.33%, but still, the experiments resulted in gestation periods and the birth of a living calf. Upon introduction of the new cell line, also the cloning protocol was optimized. TCM-199 alone was used as IVM medium and Suigonan PG600 preparation served as hormones, also, the conditions of fusion were changed: a slightly higher voltage of 70V 25µsec 2p was applied instead of the earlier voltage of 65V 25µsec 2p, because the use of a lower voltage resulted in a lower percent of the cell fusion. The most important change, however, was replacing the SOF solution of bovine serum (ES) of undefined composition used for growing the non-human embryos with the fetal bovine serum (BSA) of a more stable chemical composition, which should decrease the number of incidences of the large non-human embryo syndrome after implanting cloned non-human embryos.

## Claims

1. A method for obtaining biopharmaceuticals from transgenic bovine animals, wherein cDNA sequence of human growth hormone have been inserted into the genome of a transgenic bovine using a expression vector comprising sequence of SEQ ID No. 2, the method comprising the steps of
a. using transgenic cell lines for generating transgenic cloned embryos, wherein the primary cell lines grown from fetal bovine skin tissue serve as donor cells;
b. inserting into the selected cells of primary embryonic fibroblast cell line the expression vector plasmid into which, after the β-casein promoter, cDNA sequences encoding human growth hormone have been cloned;
c. using a neomycin G418 resistance test for selecting transgenic cells;
d. cloning the transgenic cell lines, implanting the cloned embryos, and achieving the birth of a healthy transgenic calf whose genome contains the gene of human growth hormone comprising the sequence of SEQ ID No. 5 or SEQ ID No. 6.

2. The method according to claim 1 for obtaining biopharmaceuticals from a transgenic bovine, wherein in step (b), the expression vector plasmid is incorporated into the cells of the primary embryonic fibroblast cell line and into the said plasmid, after a β-casein promoter, cDNA sequences encoding human growth hormone comprising the sequences of SEQ ID No. 5 or SEQ ID No. 6 have been cloned.

3. The method according to claim 1 for obtaining biopharmaceuticals from transgenic bovine animals, wherein in step (d), cloning is performed with transgenic cell lines, the resulting cloned embryos are implanted into a prepared recipient and consequently, the genome of a healthy transgenic calf born after the gestation period contains the genes of human growth hormone comprising the sequences of SEQ ID No. 5 or SEQ ID No. 6.

4. The method according to claim 1, wherein the concentration of the neomycine G418 resistance test used in step (c) is within the range of 600 to 900 ng/ml, more particularly 800 ng/ml.

5. A method according to claims 1 to 4 for obtaining biopharmaceuticals from transgenic bovine animals and for producing human growth hormone, human insulin or human erythropoietin from the milk of a transgenic bovine, **characterised in that** for generating a transgenic bovine, nuclear implantation, DNA nuclear microinjenction, DNA transfer with embryonic stem cells including gene targeting technology, transfer of DNA vector with viral vectors, DNA transfer with penetrating peptides, sperm-mediated transfer of DNA vectors, DNA transfer with *in vitro* and/or *in vivo* DNA transfection are used.

6. The method according to claim 5 for obtaining biopharmaceuticals from transgenic bovine animals, wherein the expression vector comprising sequence of SEQ ID No. 2 is used for expressing proteins and peptides in the milk of transgenic bovine animals.

## Patentansprüche

1. Verfahren zum Erhalt von Biopharamzeutika aus transgenen Rindern, bei dem die cDNS-Sequenz eines menschlichen Wachstumshormons in das Genom eines transgenen Rinds durch einen Expressionsvektor, bestehend aus SEQ ID Nr. 2, eingefügt wurde, wobei die 5 Verfahren die folgenden Schritte umfassen
a. verwenden von transgenen Zelllinien zur Entwicklung transgener, geklonter Embryonen, bei dem die aus fötalem Hautgewebe eines Rinds gewachsenen Primärzelllinien als Spenderzellen dienen;
b. einfügen des Plasmids des Expressionsvektors in die ausgewählten Zellen der Primärzelllinien des embryonalen Fibroblasten, nach dem β-Kasein-Promoter, in welche die cDNS-Sequenzen der verschlüsselnden, menschlichen Wachstumshormone geklont wurden;
c. verwenden einer Neomycin-G418-Widerstandsprüfung zur Auswahl transgener Zellen;
d. klonen der transgenen Zelllinien, implantieren der geklonten Embryonen, sowie Geburt eines gesunden, transgenen Kalbs, dessen Genom das Gen des menschlichen Wachstumshormons enthält, bestehend aus der Sequenz SEQ ID Nr. 5 oder SEQ ID Nr. 6.

2. Verfahren nach Anspruch I zum Erhalt von Biopharmazeutika von einem transgenen Rind, bei dem in Schritt (b) Plasmid des Expressionsvektors in die Zellen der Zelllinie des primären Fibroblasten des Embryonen und in genanntes Plasmid in das, nach dem ß-Kasein-Promotor, verschlüsselnde, menschliche Wachstumshormon der cDNS-Sequenzen, bestehend aus den Sequenzen SEQ ID Nr. 5 oder SEQ ID Nr. 6, eingesetzt wird und geklont wurde.

3. Verfahren nach Anspruch I zum Erhalt von Biopharmazeutika eines transgenen Rinds, bei dem in Schritt (d), klonen mit transgenen Zelllinien ausgeführt wird, die daraus folgenden geklonten Embryonen in einen präparierten Empfänger implantiert werden und folglich, dass der Genom des gesunden, transgenen Kalbs, welches nach der Trächtigkeitsdauer geboren wird, die Gene des menschlichen Wachstumshormons, bestehend aus den Sequenzen der SEQ ID Nr. 5 oder SEQ ID Nr. 6, enthält.

4. Verfahren nach Anspruch 1, bei dem die Konzentration in dem angewandten Neomycin G418 Widerstandstests in Schritt (c) innerhalb des Bereichs 600 bis 900 ng/ml, insbesondere 800 ng/ml, liegt.

5. Verfahren nach Anspruch I bis 4 zum Erhalt von Biopharmazeutika von einem transgenen Rind und zur Herstellung menschlichem Wachstumshormon, menschlichem Insulin oder menschlichem Erythropoietin aus der Milch eines transgenen Rinds, **gekennzeichnet dadurch, dass** zur Erzeugung eines transgenen Rinds nukleare Implantation, DNS nukleare Mikroinjektion, DNS-Übertragung mit embryonalen Stammzellen, einschließlich Verfahren zur gezielten Genmodifikation, Übertragung eines DNS-Vektors mit viralen Vektoren, DNS-Übertragung mit durchdringenden Peptiden, spermienbezogene Übertragung von DNS-Vektoren, DNS-Übertragung mit in vitro und/oder in vitro DNS-Transfektion, verwendet wird.

6. Verfahren nach Anspruch 5 zum Erhalt von Biopharmazeutika von einem transgenen Rind, bei dem der aus der Sequenz SEQ ID Nr. 2 bestehende Expressionsvektor dazu verwendet wird, Proteine und Milch von transgenen Rindern darzustellen.

## Revendications

1. Procédé d'obtention de produits biopharmaceutiques à partir de bovins transgéniques, dans lequel la séquence d'ADNc de l'hormone de croissance humaine a été insérée dans le génome d'un bovin transgénique en utilisant un vecteur d'expression comprenant la séquence de SEQ ID No. 2, le procédé comprenant les étapes suivantes :
a. Utiliser des lignées cellulaires transgéniques pour générer des embryons clonés transgéniques, dans lequel les lignées cellulaires primaires issues de tissu cutané bovin foetal servent de cellules donneuses ;
b. Insérer dans les cellules sélectionnées de la lignée cellulaire fibroblastique embryonnaire primaire du plasmide vecteur d'expression dans lequel, après des séquences d'ADNc β- promoteur caséine
l'hormone de croissance humaine codante a été clonée ;
c. Utilisant un test de résistance à la néomycine G418 pour sélectionner des cellules transgéniques ;
d. En clonant des lignées cellulaires transgéniques, en implantant des embryons clonés, et en réalisant la naissance d'un veau transgénique sain dont le génome contient le gène de l'hormone de croissance humaine comprenant la séquence de SEQ ID No. 5 ou SEQ ID No. 6.

2. Procédé selon la revendication 1 pour l'obtention de produits biopharmaceutiques à partir d'un bovin transgénique, dans lequel dans l'étape (b), le plasmide vecteur d'expression est incorporé dans les cellules de la lignée cellulaire fibroblastique embryonnaire primaire et dans ledit plasmide, après un promoteur de β-caséine, les séquences d'ADNc codant l'hormone de croissance humaine comprenant les séquences de SEQ ID No. 5 ou SEQ ID No. 6 ont été clonées.

3. Procédé selon la revendication 1 pour l'obtention de produits biopharmaceutiques à partir de bovins transgéniques, dans lequel, à l'étape (d), le clonage est effectué avec des lignées cellulaires transgéniques, les embryons clonés résultants sont implantés dans un receveur préparé et par conséquent le génome d'un veau transgénique sain après la période de gestation contient les gènes de l'hormone de croissance humaine comprenant les séquences de SEQ ID No. 5 ou SEQ ID No. 6.

4. Procédé selon la revendication 1, dans lequel la concentration du test de résistance à la néomycine G418 utilisé dans l'étape (c) est comprise dans l'intervalle de 600 à 900 ng/ml, plus particulièrement de 800 ng/ml.

5. Procédé selon les revendications 1 à 4 pour l'obtention de produits biopharmaceutiques à partir d'animaux bovins transgéniques et pour la production d'hormone de croissance humaine, d'insuline humaine ou érythropoïétine humaine issue du lait d'un bovin transgénique, **caractérisée en ce qu'**elle produit un bovin transgénique, une implantation nucléaire, une micro injonction nucléaire d'ADN, un transfert d'ADN avec des cellules souches embryonnaires comprenant une technologie de ciblage génique, un transfert d'ADN avec des peptides pénétrants , transfert de vecteurs ADN par le sperme, transfert d'ADN avec transfection d'ADN in vitro et/ou in vivo.

6. Procédé selon la revendication 5 pour l'obtention de produits biopharmaceutiques à partir de bovins transgéniques, dans lequel le vecteur d'expression comprenant la séquence de SEQ ID No. 2 est utilisé pour exprimer des protéines et des peptides dans le lait de bovins transgéniques.
